(19) Europäisches Patentamt / European Patent Office / Office européen des brevets

(11) **EP 3 547 226 A1**

(12) **EUROPEAN PATENT APPLICATION**

(43) Date of publication:
02.10.2019 Bulletin 2019/40

(51) Int Cl.:
***G06N 3/04*** *(2006.01)*

(21) Application number: **18164807.2**

(22) Date of filing: **28.03.2018**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN** | (72) Inventor: **Cheung, Patrick**<br>**5656 AE Eindhoven (NL)**<br><br>(74) Representative: **Steenbeek, Leonardus Johannes et al**<br>**Philips Intellectual Property & Standards**<br>**High Tech Campus 5**<br>**5656 AE Eindhoven (NL)** |
| (71) Applicant: **Koninklijke Philips N.V.**<br>**5656 AE Eindhoven (NL)** | |

(54) **CROSS-MODAL NEURAL NETWORKS FOR PREDICTION**

(57)　In an embodiment, at a lower neural network layer, encoded data (11) is inputted into an encoded neural network (30) to produce an encoded feature vector (14), embedded data (12) is inputted into an embedded neural network (40) to output an embedded feature vector (15), and sampled data (13) is inputted into a sampled neural network (60) (50) to output a sample feature vector (16). At an upper neural network layer, the encoded feature vector (14), the embedded feature vector (15) and the sample feature vector (16) are inputted into a convolutional neural network (60) to produce a prediction (17).

FIG. 1

EP 3 547 226 A1

**Description**

**TECHNICAL FIELD**

Various embodiments described herein relate to systems, controllers and methods for future event predictions computed by neural networks employing two or more lower layer neural networks for analyzing different data types, particularly attention-based lower layer neural networks.

**BACKGROUND**

Electronic records provide a wide range of heterogenous information about a subject, and historically traditional machine learning methods (e.g., logistic regression, decision tree, support vector machine, gradient boosting machine) have been applied to electronic records to predict an occurrence or a nonoccurrence of a future event. Recently, deep learning models of a specific form of network architecture (e.g., convolutional neural networks, recurrent neural networks) have been shown to outperform traditional machine learning models in predicting an occurrence or a nonoccurrence of a future event. However, predictive outputs of such deep learning models have been difficult to interpret, because a classifier at a final layer of a neural network processes a compact latent predictive feature representation extracted by the lower layers of the neural network that does not have an optimal architecture to process the heterogeneous information available in electronic records.

More particular to hospital/clinical readmissions, electronic medical records provide a wide range of heterogenous information in a variety of forms including, but not limited to, patient background information (e.g., demographics, social history and previous hospital/clinical readmissions), patient admission information (e.g., diagnosis, procedures, medication codes, free text from clinical notes) and patient physiological information (e.g., vital sign measurements and laboratory test results). An application of deep learning models with a specific form of neural network architecture to such electronic medical records may not generate an optimal analysis of the heterogenous information for predicting an occurrence or a nonoccurrence of a patient hospital/clinical readmission, because again a classifier at a final layer of the neural network architecture processes a compact latent predictive feature representation extracted by lower layers of the neural network architecture that does not have an optimal architecture to process the heterogeneous information.

One such known deep learning model involves (1) at a bottom layer, an extraction and sequencing of words from an electronic medical record (EMR) whereby each word is a discrete object or event (e.g., a diagnosis or a procedure) or a derived object (e.g., a time interval or a hospital transfer), (2) at a next layer, an embedding of the words in into a Euclidean space, (3) on top of the embedding layer is a convolutional neural network for generating an EMR-level feature vector based on an identification, transformation and max-pooling of predictive motifs, and (4) at a final layer, an application of classifier of the EMR-level feature vector to predict an occurrence or a nonoccurrence of a patient hospital/clinical readmission. This approach fails to generate an optimal analysis of the EMR for predicting an occurrence or a nonoccurrence of the patient hospital/clinical readmission, because the model does not have an optimal neural network architecture at the lower layers to process differing data types of information available in EMRs.

**SUMMARY**

The present disclosure addresses an ideal of neural network systems, controllers and methods for processing differing data types of information available in an electronic record (e.g., an electronic medical record) to thereby generate an optimal analysis of the electronic record for predicting an occurrence or a nonoccurrence of a future event (e.g., a patient hospital/clinical readmission). The invention is defined by the independent claims; the dependent claims define advantageous embodiments.

Embodiments provide for a partitioning of electronic data. For example, an electronic medical record may be partitioned into three (3) categories. A first category is patient background information which is not associated with any specific hospital visit (e.g., patient demographics, social history and prior hospitalizations). A second category is patient admission information associated with patient encounters in multiple hospital/clinical visits which illustrates the past history of medical conditions of the patient (e.g., structure data such as diagnosis, procedures and medication codes or unstructured such as free text from clinical notes). A third category is patient physiological information from the patient most recent hospital visit (e.g., a time series of vital sign measurements and laboratory test results).

Embodiments are premised on (1) a pre-processing of electronic data of different data types (e.g., the partitioned data categories), (2) an inputting of the pre-processed data into neural networks of different neural architectures selected for optimally extracting feature representations from the different data types and (3) combining feature vectors from the neural networks to produce a prediction whereby the prediction is based on an extracted compact predictive feature representation derived from the different data types. As such, embodiments further provide a novel and unique cross-modal neural network systems, controllers and methods for processing the partitioned electronic data. The cross-modal neural network systems, controllers and methods are based on a plurality of lower layer neural networks of different architectures to independently learn the feature representation of each category of the partitioned electronic data. The feature representations of the data from each category are then combined at a higher upper layer in order to generate a compact predictive feature representation of each category of the partitioned electronic data. Additionally, an attention module may be optionally utilized at each lower layer neural network in order to promote model interpretability.

One embodiment is a controller for processing multimodal data including a plurality of different data types. The controller comprises a processor and a non-transitory memory configured to at a lower neural network layer, at least two of (1) input a first data type into a first neural network to produce a first feature vector, (2) input a second data type into a second neural network to output a second feature vector, and (3) input a third data type into a third neural network to output an third feature vector, and at an upper neural network layer, (4) input at least two of the first feature vector, the second feature vector and the third feature vector into a fourth neural network to produce a prediction. The neural networks have different neural architectures (e.g., the neural networks include different types of neural networks or the neural networks include different versions of the same type of neural network).

A second embodiment is a controller for processing multimodal electronic data including an encoded data, an embedded data and a sampled data. The controller comprises a processor and a non-transitory memory configured to at a lower neural network layer, at least two of (1) input the encoded data into an encoded neural network to produce an encoded feature vector, (2) input the embedded data into an embedded neural network to output an embedded feature vector, and (3) input the sampled data into a sampled neural network to output an sampled feature vector, and at an upper neural network layer, (4) input at least two of the encoded feature vector, the embedded feature vector and the sampled feature vector into a convolutional neural network to produce a prediction.

A third embodiment is a non-transitory machine-readable storage medium first with instructions for execution by a processor for processing multimodal electronic data including a plurality data types. The non-transitory machine-readable storage medium comprising instructions to at a lower neural network layer, at least two of (1) input a first data type into a first neural network to output a first feature vector, (2) input a second data type into a second neural network to output a second feature vector and (3) input a third data type into a third neural network to output a third feature vector and at an upper neural network layer, (4) input at least two of the first feature vector, the second feature vector and the third feature vector into a fourth neural network to produce a prediction. The first neural network, the second neural network and the third neural network have different neural architectures (e.g., the neural networks include different types of neural networks or the neural networks include different versions of the same type of neural network).

A fourth embodiment is a non-transitory machine-readable storage medium encoded with instructions for execution by a processor for processing multimodal electronic data including an encoded data, an embedded data and a sampled data. The non-transitory machine-readable storage medium comprising instructions to at a lower neural network layer, at least two of (1) input the encoded data into an encoded neural network to output an encoded feature vector, (2) input the embedded data into an embedded neural network to output an embedded feature vector and (3) input the sampled data into a sampled neural network to output an sampled feature vector and at an upper neural network layer, (4) input at least two of the encoded feature vector, the embedded feature vector and the sampled feature vector into a convolutional neural network to produce a prediction. A fifth embodiment is a method for processing multimodal electronic data including a plurality of different data types. The method comprises, at a lower neural network layer, at least two of (1) inputting a first data type into an first neural network to output an first feature vector, (2) inputting a second data type into an second neural network to output an second feature vector and (3) inputting a third data type into a third neural network to output an third feature vector and at an upper neural network layer, (4) inputting at least two of the first feature vector, the second feature vector and the third feature vector into a fourth neural network to produce a prediction. the first neural network, the second neural network and the third neural network have different neural architectures (e.g., the neural networks include different types of neural networks or the neural networks include different versions of the same type of neural network).

A sixth embodiment is a method for processing multimodal electronic data including an encoded data, an embedded data and a sampled data. The method comprises, at a lower neural network layer, at least two of (1) inputting the encoded data into an encoded neural network to output an encoded feature vector, (2) inputting the embedded data into an embedded neural network to output an embedded feature vector and (3) inputting the sampled data into a sampled neural network to output an sampled feature vector and at an upper neural network layer, (4) inputting at least two of the encoded feature vector, the embedded feature vector and the sampled feature vector into a convolutional neural network to produce a prediction.

For purposes of describing and claiming the invention:

(1) the terms of the art of the present disclosure including, but not limited to, "electronic data", "electronic record", "pre-processing", "neural network", "deep learning network", "convolutional network", "attention module", "encoding", "embedding", "sampling", "convolution", "max pooling", "feature vector", "predictive feature representation" and "prediction", are to be broadly interpreted as known in the art and exemplary described herein;

(2) the term "encoded data" broadly encompasses electronic data encoded in accordance with neural network technology as understood in the art and hereinafter conceived. Examples of encoded data in the context of electronic medical records includes, but are not limited to, a one-hot encoding, a binary encoding and an autoencoding of categorial and numerical data informative of patient background information (e.g., demographics, social history and previous hospital/clinical readmissions);

(3) the term "encoded neural network" broadly encompasses any neural network, as understood in the art and hereinafter conceived, having an architecture exclusively designated by an embodiment for learning predictive feature representations of encoded data;

(4) the term "encoded feature vector" broadly encompasses a neural network vector representative of predictive features of encoded data as understood in the art and hereinafter conceived;

(5) the term "embedded data" broadly encompasses electronic data embedded in accordance with neural network technology as understood in the art and hereinafter conceived. Examples of embedded data in the context of electronic medical records includes, but are not limited to, a word embedding of discrete cords and words informative of patient admission information (e.g., diagnosis, procedures, medication codes and free text from clinical notes);

(6) the term "embedded neural network" broadly encompasses any neural network, as understood in the art and hereinafter conceived, having an architecture exclusively designated by an embodiment for learning feature representations of embedded data;

(7) the term "embedded feature vector" broadly encompasses a neural network vector representative of predictive features of embedded data as understood in the art and hereinafter conceived;

(8) the term "sampled data" broadly encompasses a sampling of time series data, continuous or discontinuous, as understood in the art and hereinafter conceived. Examples of sampled data in the context of electronic medical records includes, but is not limited to, a sampling of time series data informative of patient physiological information (e.g., vital sign measurements and laboratory test results);

(9) the term "sampled neural network" broadly encompasses any neural network, as understood in the art and hereinafter conceived, having an architecture exclusively designated by an embodiment for learning feature representations of sampled data;

(10) the term "sampled feature vector" broadly encompasses a neural network vector representative of predictive features of sampled data as understood in the art and hereinafter conceived;

(11) the phrase "different neural architectures" broadly encompass each neural network differing from the other neural networks by at least one structural aspect. Examples of different neural architectures include, but are not limited to, the neural networks being different types of neural networks (e.g., a deep learning network and a convolutional neural network) or the neural networks having different structural versions of the same type of neural network (e.g., a one-stage convolutional neural network and a two-stage convolutional neural network). The phrase "different neural architecture" excludes neural networks of the same type and same version configured with different parameters;

(12) the term "controller" broadly encompasses all structural configurations, as understood in the art and as exemplary described herein, of an application specific main board or an application specific integrated circuit for controlling an application of various inventive principles as subsequently described herein. The structural configuration of the controller may include, but is not limited to, processor(s), computer-usable/computer readable storage medium(s), an operating system, application module(s), peripheral device controller(s), slot(s) and port(s);

(13) the term "module" broadly encompasses electronic circuitry/hardware and/or an executable program (e.g., executable software stored on non-transitory computer readable medium(s) and/or firmware) incorporated within or accessible by a controller for executing a specific application; and

(14) the descriptive labels for term "module" herein facilitates a distinction between modules as described and claimed herein without specifying or implying any additional limitation to the term "module"; and

(15) "data" may be embodied in all forms of a detectable physical quantity or impulse (e.g., voltage, current, magnetic field strength, impedance, color) as understood in the art and as exemplary described herein for transmitting information and/or instructions in support of applying various inventive principles as subsequently described herein. Data communication encompassed by embodiments may involve any communication method as known in the art including, but not limited to, data transmission/reception over any type of wired or wireless datalink and a reading of data uploaded to a computer-usable/computer readable storage medium.

The foregoing and other embodiments as well as various features and advantages of the present disclosure will become further apparent from the following detailed description of various embodiments read in conjunction with the accompanying drawings.

**BRIEF DESCRIPTION OF THE DRAWINGS**

In order to better understand various example embodiments, reference is made to the accompanying drawings, wherein:

FIG. 1 illustrates a first exemplary embodiment of a cross-modal neural network in accordance with the present disclosure for future event predictions;

FIG. 2 illustrates an exemplary embodiment of a cross-modal neural network in accordance with the present disclosure for patient hospital/clinical readmission predictions;

FIG. 3 illustrates an exemplary embodiment of a data preprocessor in accordance with the present disclosure;

FIG. 4A illustrates an exemplary embodiment of deep neural network in accordance with the present disclosure;

FIG. 4B illustrates an exemplary embodiment of attention-based deep neural network in accordance with the present disclosure;

FIG. 5A illustrates an exemplary embodiment of a one-stage convolutional neural network in accordance with the present disclosure;

FIG. 5B illustrates an exemplary embodiment of an attention-based one-stage convolutional neural network in accordance with the present disclosure;

FIG. 6A illustrates an exemplary embodiment of a two-stage convolutional neural network in accordance with the present disclosure;

FIG. 6B illustrates an exemplary embodiment of an attention-based two-stage convolutional neural network in accordance with the present disclosure;

FIG. 7 illustrates an exemplary embodiment of a sigmoid-based convolutional neural network in accordance with the present disclosure;

FIG. 8 illustrates a cross-modal neural network system in accordance with the present disclosure;

FIG. 9 illustrates an exemplary embodiment of a cross-modal neural network controller in accordance with the present disclosure; and

FIG. 10 a second exemplary embodiment of a cross-modal neural network in accordance with the present disclosure for future event predictions.

**DETAILED DESCRIPTION**

The description and drawings presented herein illustrate various principles. It will be appreciated that those skilled in the art will be able to devise various arrangements that, although not explicitly described or shown herein, embody these principles and are included within the scope of this disclosure. As used herein, the term, "or," as used herein, refers to a non-exclusive or (*i.e.,* and/or), unless otherwise indicated (*e.g.,* "or else" or "or in the alternative"). Additionally, the various embodiments described herein are not necessarily mutually exclusive and may be combined to produce additional embodiments that incorporate the principles described herein.

Embodiments are premised on a pre-processing of different data types. For example, in the context of an electronic medical record, a first data type is patient background information which is not associated with any specific hospital visit (e.g., patient demographics, social history and prior hospitalizations), a second data type is patient admission information associated with patient encounters in multiple hospital/clinical visits which illustrates the past history of medical conditions of the patient (e.g., structure data such as diagnosis, procedures and medication codes or unstructured such as free text from clinical notes), and a third data type is patient physiological information from the patient most recent hospital visit (e.g., a time series of vital sign measurements and laboratory test results).

Embodiments are further premised on inputting of the pre-processed data into neural networks of different neural architectures selected for optimally extracting predictive feature representations from the different data types. For example, a first data type is pre-processed and inputted into a first neural network for extracting predictive feature representations from the first data type, a second data type is pre-processed and inputted into a second neural network for extracting predictive feature representations from the second data type, and a third data type is pre-processed and inputted into a third neural network for extracting predictive feature representations from the third data type, where the three (3) neural networks have different neural architectures (e.g., the neural networks include different types of neural networks or the neural networks include different versions of the same type of neural network). More particularly in the context of an electronic medical record, patent background information is encoded and inputted into an encoded neural network (e.g., a deep learning network or an attention-based deep learning network) for extracting predictive feature representations from the encoded data, patent admission information is embedded and inputted into an embedded neural network (e.g., a one-stage convolutional neural network or an attention-based one-stage convolutional neural network) for extracting predictive feature representations from the embedded data, and patient physiological information is sampled and inputted into a sampled neural network (e.g., a two-stage convolutional neural network or an attention-based two-stage convolutional neural network) for extracting predictive feature representations from the sampled data.

Embodiments are further premised on combining feature vectors from the neural networks having different neural architectures to produce a prediction whereby the prediction is based on an extracted compact predictive feature representation derived from the different data types. For example, a fourth neural network inputs a first feature vector representing predictive feature representations of a first data type, a second feature vector representing predictive feature representations of a second data type and a third feature vector representing predictive feature representations of a third data type to produce a prediction whereby the prediction is based on an extracted compact predictive feature representation derived from the different data types. More particularly in the context of an electronic medical record, a convolutional neural network (e.g., a sigmoid-based convolutional neural network) inputs a encoded feature vector representing predictive feature representations of encoded patent background information, an embedded feature vector representing predictive feature representations of embedded patent admission information and sampled feature vector representing predictive feature representations of sampled patient physiological information to produce a patient hospital/clinical readmission prediction whereby the prediction is based on an extracted compact predictive feature representation derived from the patient background information, the patient admission information and patient physiological information.

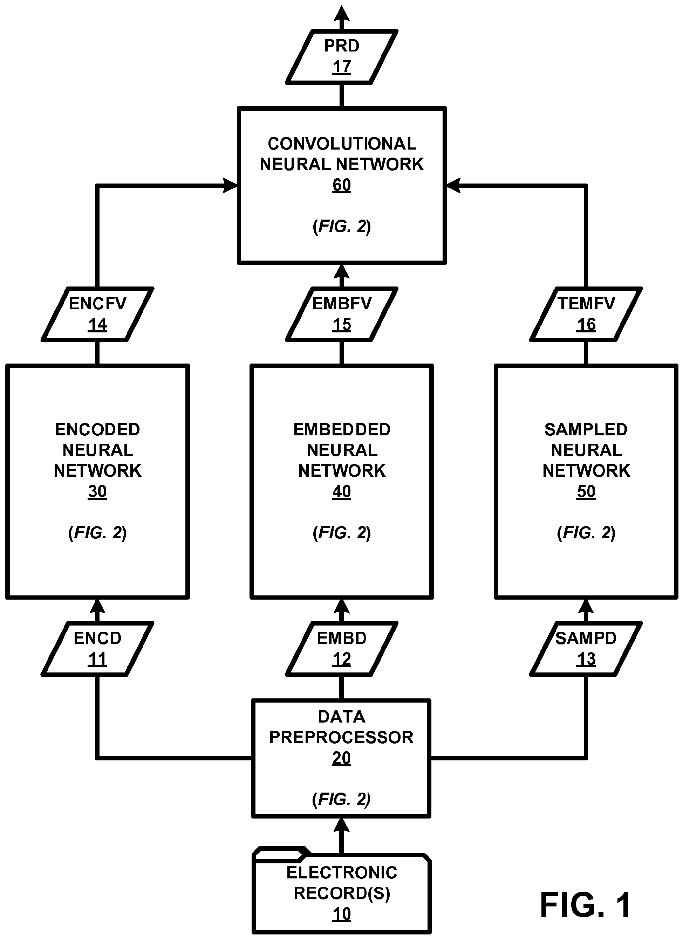

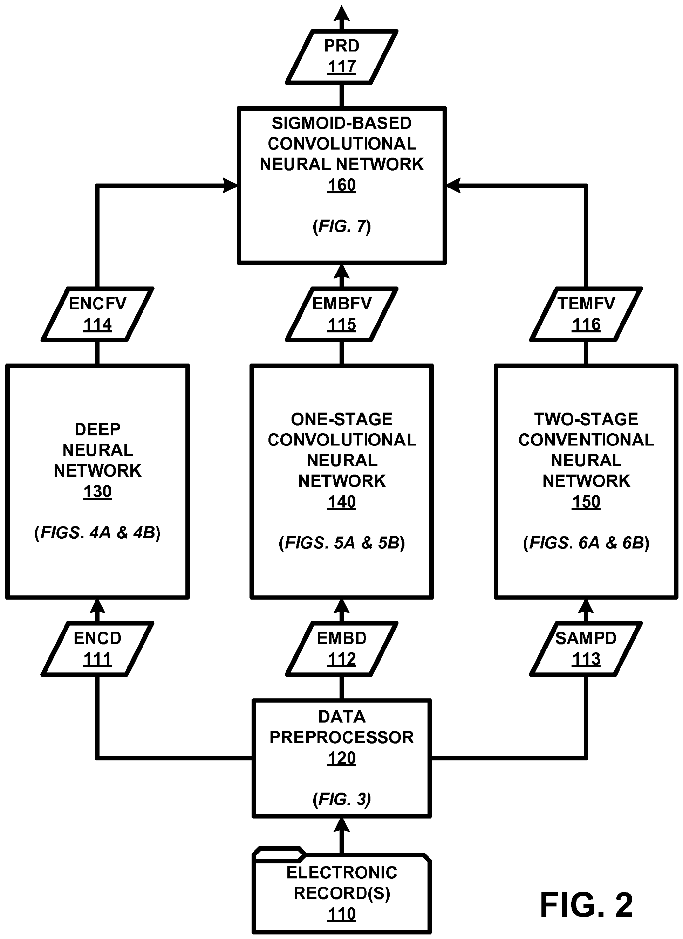

To facilitate an understanding of the embodiments, the following description of FIG. 1 teaches a cross-modal neural network for future event predictions and FIG. 2 teaches a cross-modal 1 neural network for patient hospital/clinical readmission predictions. From the description of FIGS. 1 and 2, those having ordinary skill in the art will appreciate how to apply the present disclosure for making and using numerous and various additional embodiments of cross-modal neural network of the present disclosure.

Referring to FIG. 1, a cross-modal neural network system for future event predictions employs a data preprocessor 20, an encoded neural network 30, an embedded neural network 40, a sampled neural network 50 and a convolutional neural network 60.

In operation, data preprocessor 20 is a module having an architecture for extracting different data types from electronic record(s) 10 to produce encoded data 11 form a first data type, embedded data 12 from a second data type and sampled data 13 form a third data type.

Encoded neural network 30 is a module having a neural architecture trained for analyzing encoded data 11 to learn predictive features as related to an occurrence or a nonoccurrence of a future event and inputs encoded data 11 to produce an encoded feature vector 14 representative of the predictive features of encoded data 11.

Embedded neural network 40 is a module having a neural architecture trained for analyzing embedded data 12 to learn predictive features as related to the occurrence or the nonoccurrence of the future event and inputs embedded data 12 to produce an embedded feature vector 15 representative of the predictive features of embedded data 12.

Sampled neural network 50 is a module having a neural architecture trained for analyzing sampled data 13 to learn predictive features as related to the occurrence or the nonoccurrence of the future event and inputs sampled data 13 to produce a sampled feature vector 16 representative of the predictive features of sampled data 16.

Convolutional neural network 60 is a module having a neural architecture trained for combining encoded feature vector 14, embedded feature vector 15 and sampled feature vector 16 to produce a prediction 17 of the occurrence or the nonoccurrence of the future event.

In practice, encoded data 11 is a first data type of electronic record(s) 10 encoded by the data preprocessor 20 as known in the art (e.g., one-hot coded binary coded or autoencoding), embedded data 12 is a second data type of electronic record(s) 10 embedded by the data preprocessor 20 as known in the art (e.g., a word embedding), and sampled data 13 is third data type of electronic record(s) 10 sampled by the data preprocessor 20.

Data preprocessor 20 may include a user interface for a manual loading of electronic record(s) 10 by data type or may be trained to identify the different data types of electronic record(s) 10 as known in the art.

Further in practice, in view of a neural processing different types of data, embodiments of the neural architectures of encoded neural network 30, embedded neural network 40 and sampled neural network 50 will differ by one, several or all stages of neural processing (e.g., encoded neural network 30, embedded neural network 40 and sampled neural network 50 will be different types of neural networks or encoded neural network 30, embedded neural network 40 and sampled neural network 50 will be different versions of the same type of neural network).

Exemplary neural architectures of encoded neural network 30 include, but are not limited to, a deep learning network (e.g. multilayer perceptrons).

Exemplary neural architectures of embedded neural network 40 include, but are not limited to, a one-stage convolutional network (e.g. inception architecture).

Exemplary neural architectures of sampled neural network 50 include, but are not limited to, a two-stage convolutional network (e.g. recurrent neural network).

Also in practice, the neural architectures of encoded neural network 30, embedded neural network 40 and/or sampled neural network 50 may include an attention module as known in the art.

Additionally, in practice, the neural architecture of cross-modal convolutional neural network 60 may produce prediction 17 as a binary output delineating either a predictive occurrence or a predictive nonoccurrence of the future event, or a percentage output delineating a predictive probability of an occurrence of the future event.

Exemplary neural architectures of convolutional neural network 60 include, but are not limited to, a sigmoid-based convolutional neural network (e.g. multilayer perceptrons).

Even further in practice, electronic record(s) 10 may only include two (2) of three (3) data types and therefore only the corresponding neural networks 30, 40 and 50 will be utilized, or electronic record(s) 10 may include an additional different data type whereby an additional neural network having a neural architecture different from the architectures of neural networks 30, 40 and 50 will be utilized to produce a feature vector representative of predictive features of the additional different data type.

Various embodiments of the present disclosure are directed to a deep learning model employing lower layer neural networks of different architectures to independently learn the embedded feature representation of each data type of a partitioned multimodal electronic data including encoded data 11, embedded data 12 and sampled data 13.

Referring to FIG. 2, a cross-modal neural network for patient hospital/clinical readmission predictions employs data preprocessor 20 (FIG. 1) embodied as a data preprocessor 120, encoded neural network 30 (FIG. 1) embodied as a deep neural network 130, embedded neural network 40 (FIG. 1) embodied as a one-stage convolutional neural network 140, sampled neural network 50 (FIG. 1) embodied as a two-stage convolutional neural network 150 and convolutional neural network 60 embodied as a sigmoid-based convolutional neural network 160.

In operation, data preprocessor 120 is a module for extracting encoded data 111, embedded data 112 and sampled data 113 from one or more electronic medical records 110.

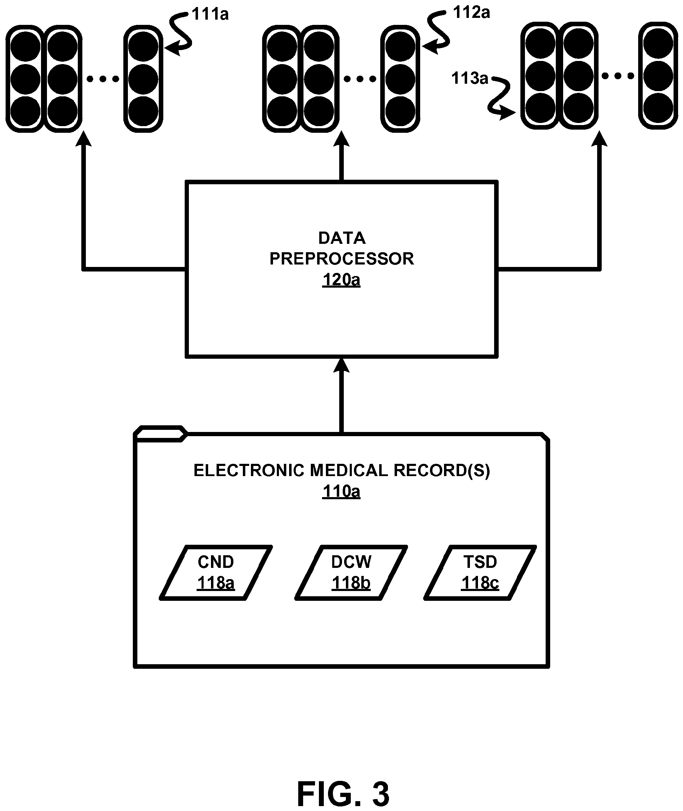

In one embodiment as shown in FIG. 3, electronic medical record(s) 110a includes categorical and numerical data 118a (e.g., demographics, social history and previous hospital/clinical admissions), discrete codes and words 118b (e.g., diagnosis, procedure and medication) and time series data 118c (e.g., vital signs and lab results).

Data preprocessor 120 extracts and encodes categorical and numerical data 118a informative of patient background information into encoded data 111a, extracts and embeds discrete codes and words 118b informative of patient admission information into embedded data 112a and extracts and samples time series data 118c informative of patient physiological information into sampled data 113a.

Deep neural network 130 is a module having a neural architecture trained for analyzing encoded data 111 to learn predictive features as related to an occurrence or a nonoccurrence of a patient hospital/clinical readmission and inputs encoded data 111 to produce an encoded feature vector 114 representative of the predictive features of encoded data 111.

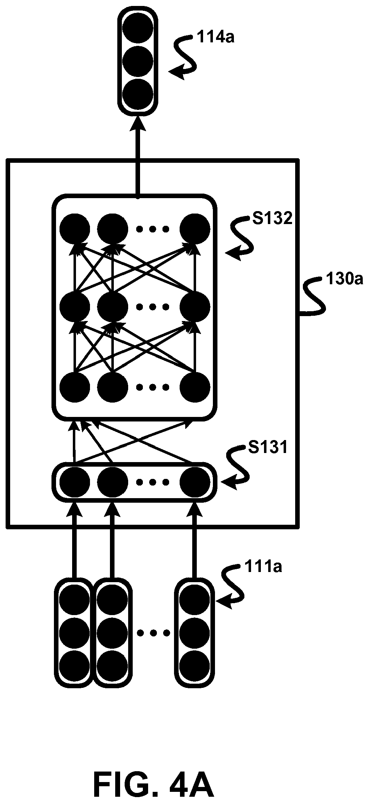

In one embodiment as shown in FIG. 4A, a deep neural network 130a has a neural architecture employing a module including a flatten stage S 131 and a deep neural network stage S132 trained to learn predictive features as related to an occurrence or a nonoccurrence of a patient hospital/clinical readmission and inputs encoded data 111a (FIG. 3) to produce an encoded feature vector 114a representative of the predictive features of encoded data 111a.

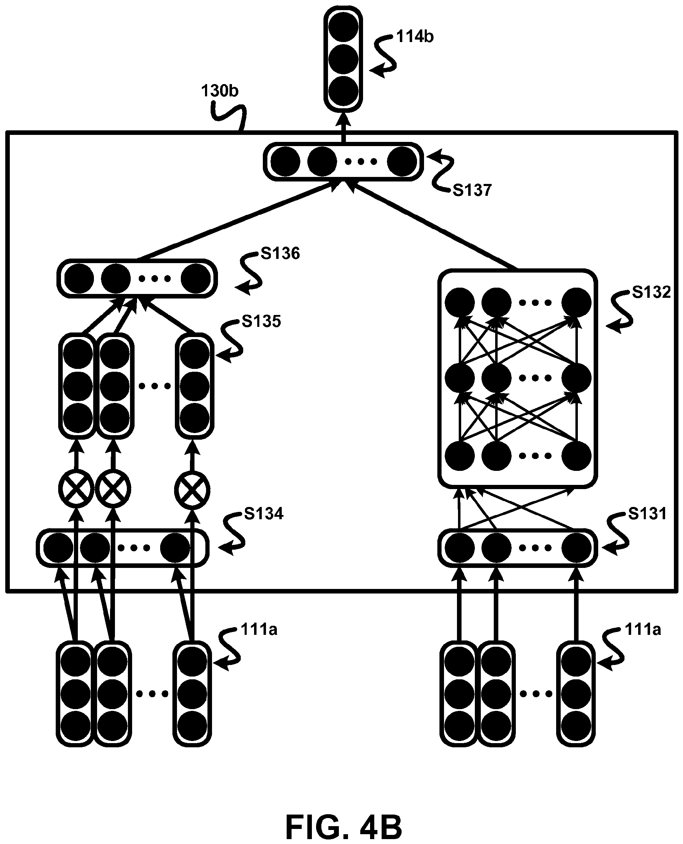

In an attention-based embodiment as shown in FIG. 4B, a deep neural network 130b has a neural architecture employing a DNN module including flatten stage S131 (FIG. 4A) and deep neural network stage S132 (FIG. 4A) trained to learn predictive features as related to an occurrence or a nonoccurrence of a patient hospital/clinical readmission and inputs encoded data 111a.

Still referring to FIG. 4B, the neural architecture of deep neural network 130b further employs an attention module including a convolution (attention) stage S134, a weighted embedded stage S135 and a summation/convolution stage S136 to produce an attention output for visualizing features of encoded data 111a that are considered to be important by the prediction model.

In practice, the architecture of the attention module is based on $\mathbf{u}_i \in \mathbb{R}^{dx1}$ as the $i$th input to deep neural network 130b where $d$ is a number of encoding bits for the background data. Convolution stage S 134 are performed on the sequence of inputs to generate an attention score $\alpha_i$ in accordance with following equations (1) and (2):

$$\mathbf{U}_{att,i} = \left( \mathbf{u}_{i+\frac{-w+1}{2}}, \mathbf{u}_{i+\frac{-w+3}{2}}, \dots, \mathbf{u}_i, \dots, \mathbf{u}_{i+\frac{w-1}{2}} \right)^T \tag{1}$$

$$\alpha_i = g(\mathbf{U}_{att,i} * \mathbf{W}_{att} + b_{att}), \tag{2}$$

where $\mathbf{W}_{att} \in \mathbb{R}^{wxd}$ is the weight matrix, * is the convolution operation, $b_{att}$ is a bias term, w is the filter length and g is the *sigmoid* activation function. Attention scores for input variables are used as weights to compute the context

vector $\mathbf{c} = \sum_i \alpha_i \mathbf{u}_i$ during stage weighted embedded S135. The context vector is then processed by a convolution stage S136 to generate the attention representation at the output of the attention module.

Still referring to FIG. 4B, the outputs of the DNN module and the attention module are then concatenated and convoluted at a stage S137 using $N_{conv}$ number of filters to produce an encoded feature vector 114b representative of the predictive features of encoded data 111a.

Referring back to FIG. 2, one-stage convolutional neural network 140 is a module having a neural architecture trained for analyzing embedded data 112 to learn predictive features as related to an occurrence or a nonoccurrence of a patient hospital/clinical readmission and inputs embedded data 112 to produce an embedded feature vector 115 representative of the predictive features of embedded data 112.

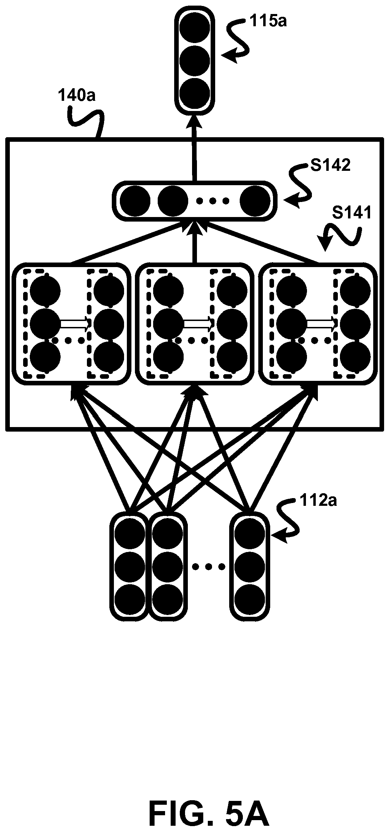

In one embodiment as shown in FIG. 5A, a one-stage convolutional neural network 140b has a neural architecture employing a module including a multiple convolutional neural network stage S141 applying convolution and max pooling with different filter widths for multi-level feature extraction and a fully connected stage S142 trained to learn predictive features of embedded data 112 (FIG. 3) as related to an occurrence or a nonoccurrence of a patient hospital/clinical readmission and inputs embedded data 112a to produce an embedded feature vector 115a representative of the predictive features of embedded data 112a.

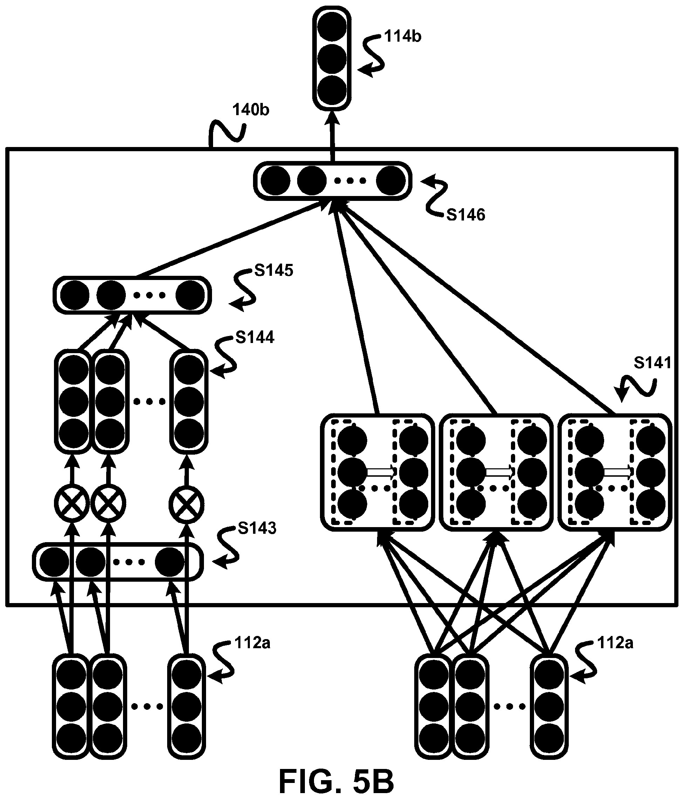

In an attention-based embodiment as shown in FIG. 5B, a one-stage convolutional neural network 140b employs a convolutional module including multiple convolutional neural network stage S141 (FIG. 5A) applying convolution and max pooling with different filter widths for multi-level feature extraction trained to learn predictive features embedded data 112 (FIG. 3) as related to an occurrence or a nonoccurrence of a patient hospital/clinical readmission.

Still referring to FIG. 5B, the neural architecture of one-stage convolutional neural network 140b further employs an attention module including a convolution (attention) stage S143, a weighted embedded stage S144 and a summation/convolution stage S145 to produce an attention output for visualizing features of embedded data 112a that are considered to be important by the prediction model.

In practice, the architecture of the attention module is based on $\mathbf{u}_i \in \mathbb{R}^{d\mathrm{x}1}$ as the $i^{\text{th}}$ input to one-stage convolutional neural network 140b where $d$ is a word embedding dimension of discrete medical codes. Convolution stage S143 is performed on the sequence of inputs to generate an attention score $\alpha_i$ in accordance with following equations (1) and (2):

$$\mathbf{U}_{att,i} = \left(\mathbf{u}_{i+\frac{-w+1}{2}}, \mathbf{u}_{i+\frac{-w+3}{2}}, \ldots, \mathbf{u}_i, \ldots, \mathbf{u}_{i+\frac{w-1}{2}}\right)^T \tag{1}$$

$$\alpha_i = g(\mathbf{U}_{att,i} * \mathbf{W}_{att} + b_{att}), \tag{2}$$

where $\mathbf{W}_{att} \in \mathbb{R}^{w\mathrm{x}d}$ is the weight matrix, * is the convolution operation, $b_{att}$ is a bias term, w is the filter length and $g$ is the *sigmoid* activation function. Attention scores for input variables are used as weights to compute the context vector $\mathbf{c} = \sum_i \alpha_i \mathbf{u}_i$ during stage S144. The context vector is then processed by a second convolution stage S145 to generate the attention representation at the output of the attention module.

Still referring to FIG. 5B, the outputs of the convolutional module and the attention module are then concatenated and convoluted at a stage S146 using $N_{conv}$ number of filters to produce an embedded feature vector 115b representative of the predictive features of embedded data 112a.

Referring back to FIG. 2, two-stage convolutional neural network 150 is a module having a neural architecture trained for analyzing sampled data 113 to learn predictive features as related to an occurrence or a nonoccurrence of a patient hospital/clinical readmission and inputs sampled data 113 to produce a sampled feature vector 116 representative of the predictive features of sampled data 113.

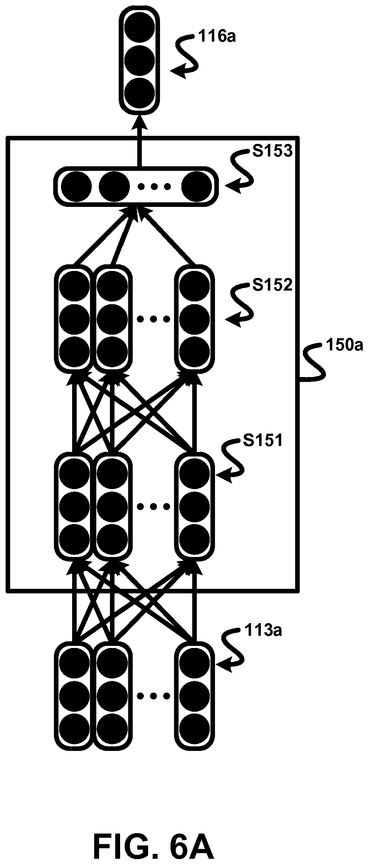

In one embodiment as shown in FIG. 6A, a two-stage convolutional neural network 150b has a neural architecture employing a module including two stacked convolutional neural network stages S151 and S152 applying convolution and max pooling for multi-level feature extraction and a fully connected stage S153 trained to learn predictive features sampled data 113 (FIG. 3) as related to an occurrence or a nonoccurrence of a patient hospital/clinical readmission and inputs sampled data 113a to produce a sampled feature vector 116a representative of the predictive features of sampled data 113a.

More particularly, each time series is considered as a channel input whereby the stage S 151 and S152 is denoted as *C1(Size)-S1-C2(Size)-S2* where *C1* and *C2* are the numbers of convolutional filters in stages S151 and S152, *Size* is the kernel size and *S1* and *S2* are subsampling factors.

Subsampling is implemented by a max pooling operation and subsampling factors are chosen such that a maximum value is obtained for each filter after stage S152.

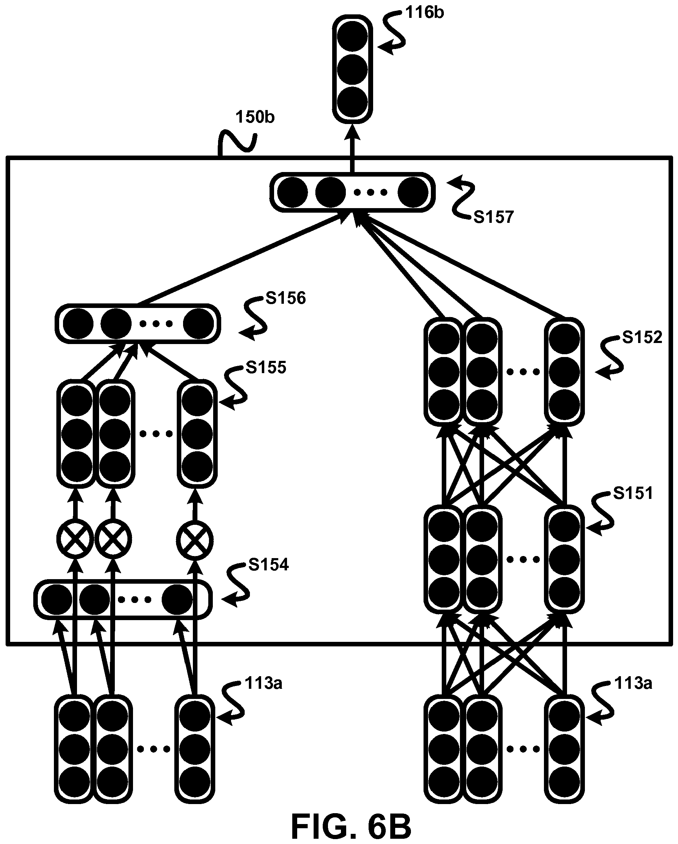

In an attention-based embodiment as shown in FIG. 6B, a two-stage convolutional neural network 150b employs a convolutional module including two stacked convolutional neural network stages S151 and S152 (FIG. 5A) applying convolution and max pooling for multi-level feature extraction trained to learn predictive features sampled data 113 (FIG. 3) as related to an occurrence or a nonoccurrence of a patient hospital/clinical readmission.

Again, each time series is considered as a channel input whereby the stage S 151 and S152 is denoted as *C1(Size)-S1-C2(Size)-S2* where *C1* and *C2* are the numbers of convolutional filters in stages S 151 and S152, *Size* is the kernel size and *S1* and *S2* are subsampling factors. Subsampling is implemented by a max pooling operation and subsampling factors are chosen such that a maximum value is obtained for each filter after stage S152.

Still referring to FIG. 6B, the neural architecture of two-stage convolutional neural network 150b further employs an attention module including a convolution (attention) stage S154, a weighted embedded stage S155 and a summation/convolution stage S156 to produce an attention output for visualizing features of sampled data 113a that are considered to be important by the prediction model.

In practice, the architecture of the attention module is based on $\mathbf{u}_i \in \mathbb{R}^{d \times 1}$ as the $i^{th}$ input to two-stage convolutional neural network 150b where $d$ is a number of data points in a time-series. Convolution stage S154 is performed on the sequence of inputs to generate an attention score $\alpha_i$ in accordance with following equations (1) and (2):

$$\mathbf{U}_{att,i} = \left(\mathbf{u}_{i+\frac{-w+1}{2}}, \mathbf{u}_{i+\frac{-w+3}{2}}, \dots, \mathbf{u}_i, \dots, \mathbf{u}_{i+\frac{w-1}{2}}\right)^T \tag{1}$$

$$\alpha_i = g(\mathbf{U}_{att,i} * \mathbf{W}_{att} + b_{att}), \tag{2}$$

where $\mathbf{W}_{att} \in \mathbb{R}^{w \times d}$ is the weight matrix, * is the convolution operation, $b_{att}$ is a bias term, w is the filter length and $g$ is the *sigmoid* activation function. Attention scores for input variables are used as weights to compute the context vector $\mathbf{c} = \sum_i \alpha_i \mathbf{u}_i$ during stage S155. The context vector is then processed by a second convolution stage S156 to generate the attention representation at the output of the attention module.

Still referring to FIG. 6B, the outputs of the convolution module and the attention module are then concatenated and convoluted at a stage S157 using $N_{conv}$ number of filters to produce a sampled feature vector 116b representative of the predictive features of sampled data 113a Referring back to FIG. 2, convolutional neural network 160 is a module having a neural architecture trained for combining encoded feature vector 114, embedded feature vector 115 and sampled feature vector 116 to produce a prediction 117 of the occurrence or the nonoccurrence of a patient hospital/clinical readmission.

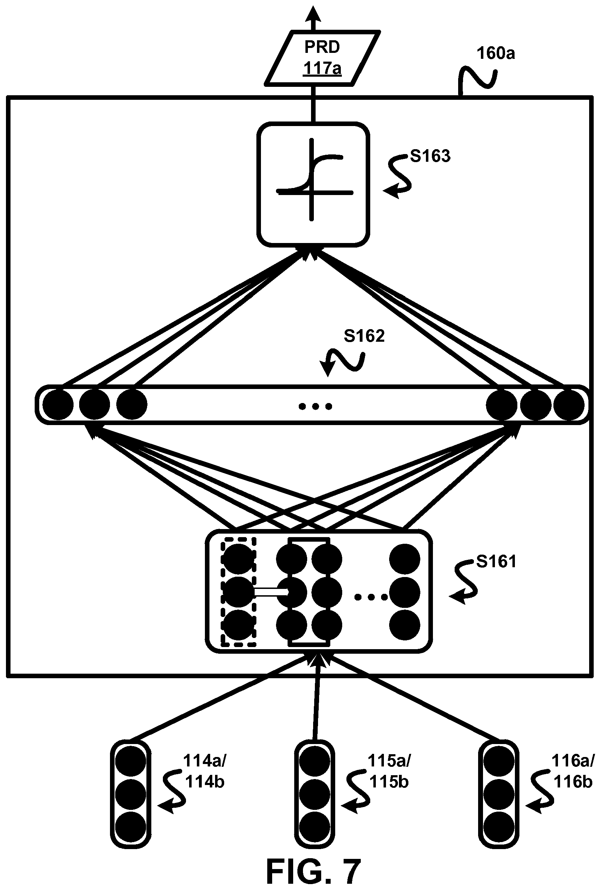

In one embodiment as shown in FIG. 7, cross-modal convolutional neural network 160a has a neural architecture employing a module including a convolutional stage S161, a fully connected stage S162 and a sigmoid function stage S163 trained to combine encoded feature vector 114a (FIG. 4A) or 114b (FIG. 4B), embedded feature vector 115a (FIG. 5A) or vector 115b (FIG. 5B) and sampled feature vector 116a (FIG. 6A) or vector 116b (FIG. 6B) to produce a prediction 117a of the occurrence or the nonoccurrence of a patient hospital/clinical readmission.

In practice, cross-modal convolutional neural network 160a is based on $\mathbf{x}_k \in \mathbb{R}^{N_{conv} \times 1}$ being a feature vector for the $k^{th}$ EMR category. The feature vectors 114, 115 and 116 from $K = 3$ modules are concatenated and convoluted at stage S161 with a matrix $\mathbf{W} \in \mathbb{R}^{K \times N_{xconv}}$ and a bias $\mathbf{b} \in \mathbb{R}^{N_{xconv} \times 1}$ in accordance with the following equations (3), (4) and (5):

$$\mathbf{X} = (\mathbf{x}_1, \mathbf{x}_2, \dots, \mathbf{x}_K)^T, \tag{3}$$

$$\mathbf{Y}(i,j) = f\big(\mathbf{X}(:,i) * \mathbf{W}(:,j) + \mathbf{b}(j)\big) \tag{4}$$

$$i \in [1, N_{conv}], \qquad j \in [1, N_{xconv}] \tag{5}$$

where $N_{xconv}$ is the number of filters and $f$ is a non-linear activation function. A max pooling operation is applied to each filter to extract a scalar $y(j) = MAX(\mathbf{Y}(:,j))$. Scalars from $N_{xconv}$ filters are then concatenated to form a compact predictive feature vector which is then fed to a final connected network at stage S162 followed by a *sigmoid* function at stage S163 to produce prediction 117a.

To facilitate a further understanding of the invention, the following is a description of an exemplary implementation of an attention-based cross-modal neural network (AXCNN) that in practice employs deep neural network 130b (FIG. 4B), one-stage convolutional neural network 140b (FIG. 5B), two-stage convolutional neural network 150b (FIG. 6B) and sigmoid-based convolutional neural network 160a (FIG. 7).

Data Pre-Processing.

The AXCNN was applied to a 30-day unplanned readmission data for heart failure (HF) collected from a large hospital system in Arizona, United States. The dataset consisted of patient encounter information for 6730 HF patients of age 18 or over (mean: 72.7, std: 14.4), 60% are males, between October 2015 and June 2017. Among them 853 patients have at least a readmission within 30 days after discharge which gives about 13% of the unplanned HF readmission rate. For each patient, the last hospital visit was identified in which the patient was diagnosed with heart failure among multiple visits and the AXCNN was used to predict if the HF patient would be readmitted within the next 30 days. The following Table I shows the summary statistics of the dataset.

TABLE I SUMMARY STATISTICS OF THE EMR DATASET

| No. of patients | 6730 | No. of medical codes (D: diagnosis, P: procedure, M: medication) | 1928 (level 3 D: 813, level 3 P: 127, M: 988) |
|---|---|---|---|
| No. of visits | 11804 | No. of medical codes per visit | 46.76 (mean), 128 (max) |
| Avg. no. of visits per patient | 1.75 | No. of data points per vital sign | 126.62 (mean), 10571 (max) |
| % of no. of visits > 3 | 8.7% | No. of data points per lab test results | 28.1 (mean), 2641 (max) |

From the EMRs, 19 background variables were selected from the patient demographics, social history and prior hospitalizations (e.g. race, tobacco use, number of prior inpatient admissions) as the input to deep neural network 130b (FIG. 4B). Unknown is assigned to missing data for nominal variables and 0 for ordinal and count variables. For the one-stage convolutional neural network 140b (FIG. 5B), level 3 ICD-10 CM and PCS codes were collected for diagnosis and procedure codes respectively, and order catalog codes for medications for each patient's encounters in the dataset up to and include the most recent hospital visit. The codes were transformed into a sentence with a time-gap word (e.g. 0-1m for 0 to 1 month interval gap) inserted between two consecutive visits and assign any codes that appear only once in the dataset to the code word rareword for robustness. The sequence was then truncated to keep the last 100 words which results in 75% of the sequences remain to be conserved. For the two-stage convolutional neural network 150b (FIG. 6B), five vital sign measurements (respiratory rate, systolic blood pressure, heart rate, blood oxygen saturation (SpO2) and temperature) and five laboratory test results (sodium, potassium, blood urea nitrogen (BUN), creatinine and the ratio of BUN to creatinine) were extracted from the last encounter before prediction for each patient. Each time series was normalized by z-score across all patients and any patient laboratory test results that are not found in EMRs are set to zeros. Furthermore, vital sign measurements and laboratory test results were resampled with backward-filled for every hour and every 4 hours respectively based on the hospital system's adult standard of care policy. Those resampled time series which have more than 100 points were truncated to keep the last 100 measurement points while others which have fewer than 100 points were zero-padded to maintain 100 points in length.

After preprocessing, the dataset was randomly divided into training (70%), validation (10%) and test sets (20%), with each set containing the same ratio of readmitted to non-readmitted patients. A validation set was used to fine tune the following hyper-parameters: number of layers in DNN 130a, number of neurons per layer in DNN 130a and FC layers, number of convolutional filters and the dropout probability.

Parameter Setting.

For the DNN in network 130b (FIG. 4B), 3 hidden layers with 64 neurons per layer were chosen. For network 140b (FG. 5B), the discrete medical codes in the sentence were embedded using the word2vec skip-gram model with the embedding dimension set to 100. The three filter widths were set to 3, 4 and 5 with 20 filters each for CNN. For the MC-DCNN in network 150b (FIG. 6B), *C1(Size)-S1-C2(Size)-S2* equal to 10(5)-10-5(5)-10 were set. For the attention modules, the filter length w = 5 was used for the first convolutional layer to generate the attention scores and 10 filters for the second convolutional layer with *tanh* to generate the attention representation. $N_{conv}$ = 20 was selected for the convolutional layer of network 160a (FIG. 7) located at the output of networks 130b, 140b and 150b. For the final layer of network 160a, $N_{xconv}$= 50 was chosen for the cross-modal convolutional layer with *relu* and 256 neurons for the FC layer. Dropouts with probability of 0.4 were utilized at the outputs of hidden layers in DNN and both the inputs and outputs of FC during training.

Implementation.

The AXCNN was implemented using a deep learning library utilizing an Adadelta optimizer with the default parameter values and batch size of 256 for training the model. Binary cross-entropy was used as the loss function to adjust the weights. Training was stopped when no further improvement on the validation loss is found after 25 epochs. The results provided an improvement over the prior art.

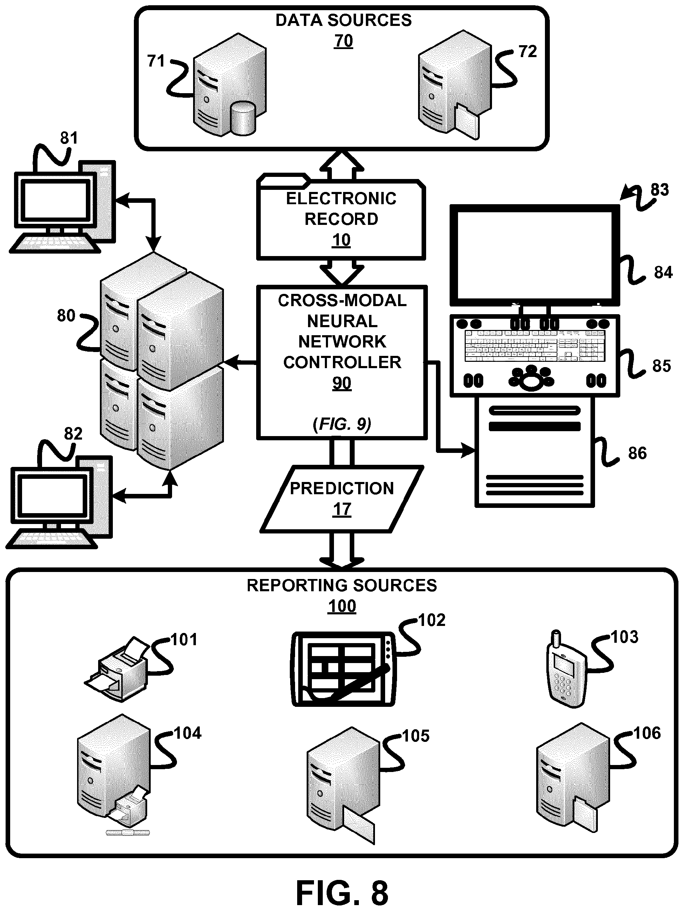

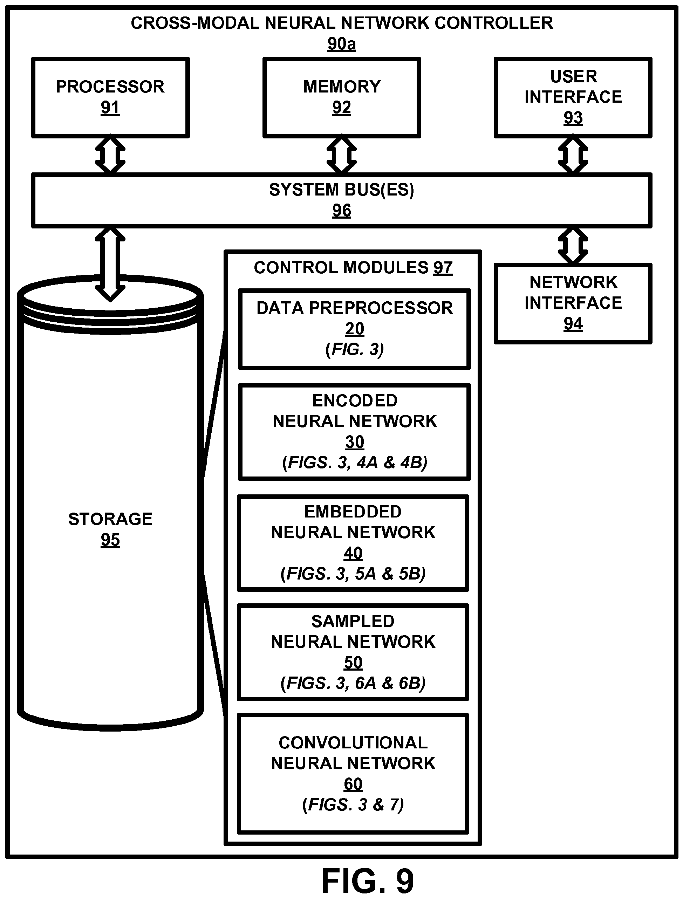

To facilitate a further understanding of the invention, the following description of FIG. 8 teaches an embodiment of a cross-modal neural network system and FIG. 9 teaches a cross-modal neural network controller. From the description of FIGS. 8 and 9, those having ordinary skill in the art will appreciate how to apply the present disclosure for making and using numerous and various additional embodiments of cross-modal neural network systems and cross-modal neural network controllers.

Referring to FIG. 8, a cross-modal neural network controller 90 is installed within an application server 80 accessible by a plurality of clients (e.g., a client 81 and a client 82 as shown) and/or is installed within a workstation 83 employing a monitor 84, a keyboard 85 and a computer 86.

In operation, cross-modal convolutional neural network 90 inputs electronic record(s) 10 (FIG. 1) from one or more data sources 70 (e.g., a database server 71 and a file server 72) to produce prediction 17 (FIG. 1) as previously described herein. Prediction 17 is communicated by controller 90 to a variety of reporting sources including, but not limited to, a printer 101, a tablet 102, a mobile phone 103, a print server 104, an email server 105 and a file server 106.

In practice, cross-modal convolutional neural network 90 may be implemented as hardware/circuity/software/firmware.

In one embodiment as shown in FIG. 9, a cross-modal convolutional neural network 90a includes a processor 91, a memory 92, a user interface 93, a network interface 94, and a storage 95 interconnected via one or more system bus(es) 96. In practice, the actual organization of the components 91-95 of controller 90a may be more complex than illustrated.

The processor 91 may be any hardware device capable of executing instructions stored in memory or storage or otherwise processing data. As such, the processor 91 may include a microprocessor, field programmable gate array (FPGA), application-specific integrated circuit (ASIC), or other similar devices.

The memory 92 may include various memories such as, for example L1, L2, or L3 cache or system memory. As such, the memory 92 may include static random access memory (SRAM), dynamic RAM (DRAM), flash memory, read only memory (ROM), or other similar memory devices.

The user interface 93 may include one or more devices for enabling communication with a user such as an administrator. For example, the user interface 93 may include a display, a mouse, and a keyboard for receiving user commands. In some embodiments, the user interface 93 may include a command line interface or graphical user interface that may be presented to a remote terminal via the network interface 94.

The network interface 94 may include one or more devices for enabling communication with other hardware devices. For example, the network interface 94 may include a network interface card (NIC) configured to communicate according to the Ethernet protocol. Additionally, the network interface 94 may implement a TCP/IP stack for communication according to the TCP/IP protocols. Various alternative or additional hardware or configurations for the network interface will be apparent.

The storage 95 may include one or more machine-readable storage media such as read-only memory (ROM), random-access memory (RAM), magnetic disk storage media, optical storage media, flash-memory devices, or similar storage media. In various embodiments, the storage 95 may store instructions for execution by the processor 91 or data upon with the processor 91 may operate. For example, the storage 95 store a base operating system (not shown) for controlling various basic operations of the hardware.

More particular, storage 95 further stores control modules 97 including an embodiment of data preprocessor 20 (e.g., data processor 102a of FIG. 3), an embodiment of encoded neural network 30 (e.g., deep learning network 130a of FIG. 4A, or attention-based deep learning network 130b of FIG. 4B), an embodiment of embedded neural network 40 (e.g., one-stage convolutional neural network 140a of FIG. 5A, or attention-based one-stage convolutional neural network 140b of FIG. 5B), an embodiment of sampled neural network 50 (e.g., two-stage convolutional neural network 150a of FIG. 6A, or attention-based two-stage convolutional neural network 150b of FIG. 6B) and an embodiment of convolutional neural network 60 (e.g., sigmoid-based convolution neural network 160a of FIG. 7).

Referring back to FIG. 1, to facilitate an understanding of the invention, the embodiments described herein were directed to three (3) specific pre-processing techniques of electronic data including encoding (e.g., encoding of categorical and numerical data), embedding (e.g., embedding of discrete codes and words) and sampling (e.g., sampling of time series data). These pre-processing techniques were chosen in view of embodiments of pre-processing categorial and numerical data, discrete codes and words and time series data as different data types of electronic medical records. Nonetheless, in practice, electronic records, particularly electronic medical records, may include additional types of data different from categorial and numerical data, discrete codes and words and time series data. As stated earlier herein, embodiments are premised on (1) pre-processing of electronic data of different data types, (2) an inputting of the pre-processed data into neural networks of different neural architectures selected for optimally extracting feature representations from the different data types and (3) combining feature vectors from the neural networks to produce a prediction whereby the prediction is based on an extracted compact predictive feature representation derived from the different data types. Thus, the claims should not be limited to embodiments of encoded neural networks, embedded neural networks, sampled neural networks unless a claim explicitly recites an encoded neural network, an embedded neural network and/or a sampled neural network.

Referring to FIGS. 1-9, those having ordinary skill in the art will appreciate the many benefits of embodiments including, but not limited to, a cross-modal neural network that addresses compact latent predictive feature representation as known in the art by extracting a compact predictive feature representation derived from the different data types.

More particularly, those having ordinary skill in the art will appreciate that embodiments are premised on a pre-processing of different data types.

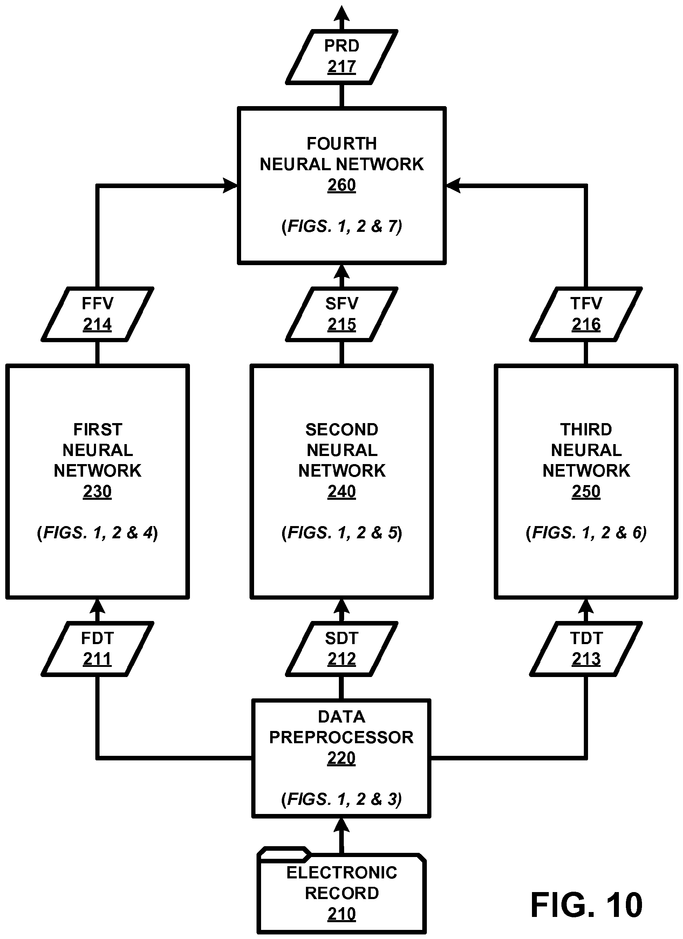

For example, FIG. 10 illustrate an electronic record 10 including first data type 211, a second data type 212 and a third data type 213. In the context of electronic record 10 being an electronic medical record, a first data type 211 may be patient background information which is not associated with any specific hospital visit (e.g., patient demographics, social history and prior hospitalizations), a second data type 212 may be patient admission information associated with patient encounters in multiple hospital/clinical visits which illustrates the past history of medical conditions of the patient (e.g., structure data such as diagnosis, procedures and medication codes or unstructured such as free text from clinical notes), and third data type 213 may be patient physiological information from the patient most recent hospital visit (e.g., a time series of vital sign measurements and laboratory test results).

Still referring to FIG. 10, first data type 211 is pre-processed by a data pre-processor 220 and inputted into a first neural network 23- for extracting predictive feature representations from first data type 211 to produce a first feature vector 214, second data type 212 is pre-processed by data pre-processor 220 and inputted into a second neural network 240 for extracting predictive feature representations from second data type 212 to produce a second feature vector 215, and third data type 213 is pre-processed by data pre-processor 220 and inputted into a third neural network 250 for extracting predictive feature representations from third data type 213 to produce a third feature vector 216, where the three (3) neural networks 230, 240 and 250 have different neural architectures (e.g., the neural networks include different types of neural networks or the neural networks include different versions of the same type of neural network).

Still referring to FIG. 10, a fourth neural network 260 combines feature vectors 214, 215, 216 from the neural networks 230, 240, 250 to produce a prediction whereby the prediction 217 that is based on an extracted compact predictive feature representation derived from the different data types 211, 212 and 213.

Furthermore, it will be apparent that various information described as stored in the storage may be additionally or alternatively stored in the memory. In this respect, the memory may also be considered to constitute a "storage device" and the storage may be considered a "memory." Various other arrangements will be apparent. Further, the memory and storage may both be considered to be "non-transitory machine-readable media." As used herein, the term "non-transitory" will be understood to exclude transitory signals but to include all forms of storage, including both volatile and non-volatile memories.

While the device is shown as including one of each described component, the various components may be duplicated in various embodiments. For example, the processor may include multiple microprocessors that are configured to independently execute the methods described herein or are configured to perform steps or subroutines of the methods described herein such that the multiple processors cooperate to achieve the functionality described herein. Further, where the device is implemented in a cloud computing system, the various hardware components may belong to separate physical systems. For example, the processor may include a first processor in a first server and a second processor in a second server.

It should be apparent from the foregoing description that various example embodiments of the invention may be implemented in hardware or firmware. Furthermore, various exemplary embodiments may be implemented as instructions stored on a machine-readable storage medium, which may be read and executed by at least one processor to perform the operations described in detail herein. A machine-readable storage medium may include any mechanism for storing information in a form readable by a machine, such as a personal or laptop computer, a server, or other computing device. Thus, a machine-readable storage medium may include read-only memory (ROM), random-access memory (RAM), magnetic disk storage media, optical storage media, flash-memory devices, and similar storage media.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in machine readable media and so executed by a computer or processor, whether or not such computer or processor is explicitly shown.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the scope of the invention. Accordingly, the foregoing disclosure, description, and figures are for illustrative purposes only and do not in any way limit the invention, which is defined only by the independent claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims that do not refer to one another may advantageously be combined.

## Claims

1.   A cross-modal neural network controller (90) for processing multimodal electronic data including a plurality of different data types, the cross-modal neural network controller (90) comprising a processor (91) and a non-transitory memory (92) configured to:

   at a lower neural network layer, at least two of:

   input a first data type (211) into a first neural network (230) to produce a first feature vector (214),
   input a second data type (212) into a second neural network (240) to output a second feature vector (215), and
   input a third data type (213) into a third neural network (250) to output a third feature vector,
   wherein the first neural network (230), the second neural network (240) and the third neural network (250) have different neural architectures; and

   at an upper neural network layer, input of the at least two of the first feature vector (214), the second feature vector (215) and the third feature vector (216) into a fourth neural network (260) to produce a prediction (217).

2.   The cross-modal neural network controller (90) of claim 1,
   wherein the first data type (211) is encoded data (11);
   wherein the input of the first data type (211) into the first neural network (230) to produce the first feature vector (214) includes:
   an input of the encoded data (11) into an encoded neural network (30) to produce an encoded feature vector (14); and
   wherein the input of the at least two of the first feature vector (214), the second feature vector (215) and the third feature vector (216) into the fourth neural network (260) to produce the prediction (217) includes:
   an input of at least two of the encoded feature vector (14), the second feature vector (215) and the third feature vector (216) into a convolutional neural network to produce the prediction (217).

3.   The cross-modal neural network controller (90) of claim 1, wherein the input of the first data type (211) into the first neural network (230) to produce the first feature vector (214) includes:

   an application of a deep learning network to the first data type (211) to generate the first feature vector (214); or
   a convolution of an application of the deep learning network and an attention module to the first data type (211) to generate the first feature vector (214).

4.   The cross-modal neural network controller (90) of claim 1,
   wherein the second data type (212) is embedded data (12);
   wherein the input of the second data type (212) into the second neural network (240) to produce the second feature vector (215) includes:
   an input of the embedded data (12) into an embedded neural network (40) to produce an embedded feature vector (15); and
wherein the input of the at least two of the first feature vector (214), the second feature vector (215) and the third feature vector (216) into the fourth neural network (260) to produce the prediction (217) includes:
an input of at least two of the first feature vector (214), the embedded feature vector (15) and the third feature vector (216) into the fourth neural network (260) to produce the prediction (217).

5. The cross-modal neural network controller (90) of claim 1, wherein the input of the second data type (212) into the second neural network (240) to produce the second feature vector (215) includes:

> an application of a one-stage convolutional neural network to the second data type (212) to generate the second feature vector (215); or
> a convolution of an application of the one-stage convolutional neural and an attention module to the second data type (212) to generate the second feature vector (215).

6. The cross-modal neural network controller (90) of claim 1,
wherein the third data type (213) is sampled data (13);
wherein the input of the third data type (213) into the third neural network (250) to produce the third feature vector (216) includes:
an input of the sampled data (13) into a sampled neural network (60) to produce a sample feature vector (16); and
wherein the input of the at least two of the first feature vector (214), the second feature vector (215) and the third feature vector (216) into the fourth neural network (260) to produce the prediction (217) includes:
an input of at least two of the first feature vector (214), the second feature vector (215) and the sample feature vector (16) into a convolutional neural network to produce the prediction (217).

7. The cross-modal neural network controller (90) of claim 1, wherein the input of the third data type (213) into the third neural network (250) to produce the third feature vector (216) includes:

> an application of a two-stage convolutional neural to the third data type (213) to generate the third feature vector (216); or
> a convolution of an application of the two-stage convolutional neural and an attention module to the third data type (213) to generate the third feature vector (216).

8. The cross-modal neural network controller (90) of claim 1, wherein the processor (91) and the non-transitory memory (92) are at least one of installed in and linked to at least one of a server, a client and a workstation.

9. A method for processing multimodal electronic data including an encoded data (11), an embedded data (12) and a sampled data (13),
the method comprising:

> at a lower neural network layer, at least two of:

>> inputting a first data type (211) into a first neural network (230) to produce a first feature vector (214),
>> inputting a second data type (212) into a second neural network (240) to output a second feature vector (215), and
>> inputting a third data type (213) into a third neural network (250) to output a third feature vector,
>> wherein the first neural network (230), the second neural network (240) and the third neural network (250) have different neural architectures; and

> at an upper neural network layer, inputting at least two of the first feature vector (214), the second feature vector (215) and the third feature vector (216) into a fourth neural network (260) to produce a prediction (217).

10. The method of claim 9,
wherein the first data type (211) is encoded data (11);
wherein the inputting of the first data type (211) into the first neural network (230) to produce the first feature vector (214) includes:

> inputting the encoded data (11) into an encoded neural network (30) to produce an encoded feature vector (14);
> wherein the inputting of the at least two of the first feature vector (214), the second feature vector (215) and the third feature vector (216) into the fourth neural network (260) to produce the prediction (217) includes:
inputting at least two of the encoded feature vector (14), the second feature vector (215) and the third feature vector (216) into a convolutional neural network to produce the prediction (217).

11. The method of claim 9,
wherein the second data type (212) is embedded data (12);
wherein the inputting of the second data type (212) into the second neural network (240) to produce the second feature vector (215) includes:

inputting the embedded data (12) into an embedded neural network (40) to produce an embedded feature vector (15); and
wherein the inputting of at least two of the first feature vector (214), the second feature vector (215) and the third feature vector (216) into the fourth neural network (260) to produce the prediction (217) includes:
inputting at least two of the first feature vector (214), the embedded feature vector (15) and the third feature vector (216) into the fourth neural network (260) to produce the prediction (217).

12. The method of claim 9,
wherein the third data type (213) is sampled data (13);
wherein the inputting of the third data type (213) into the third neural network (250) to produce the third feature vector (216) includes:
inputting the sampled data (13) into a sampled neural network (60) to produce a sample feature vector (16); and
wherein the inputting of at least two of the first feature vector (214), the second feature vector (215) and the third feature vector (216) into the fourth neural network (260) to produce the prediction (217) includes:
inputting at least two of the first feature vector (214), the second feature vector (215) and the sample feature vector (16) into a convolutional neural network to produce the prediction (217).

13. The method of claim 9, wherein the inputting of the at least two of the first feature vector (214), the second feature vector (215) and the third feature vector (216) into the fourth neural network (260) to produce the prediction (217) includes:

applying a sigmoid function to a convolving and a max pooling of the at least two of the first feature vector (214), the second feature vector (215) and the third feature vector (216).

14. A non-transitory machine-readable storage medium encoded with instructions for execution by a processor (91) for processing multimodal electronic data including an encoded data (11), an embedded data (12) and a sampled data (13), the non-transitory machine-readable storage medium comprising instructions to perform the method of any of claims 9 - 13.

**FIG. 1**

FIG. 2

**FIG. 3**

FIG. 4A

FIG. 4B

**FIG. 5A**

**FIG. 5B**

**FIG. 6A**

FIG. 6B

FIG. 7

**FIG. 8**

CROSS-MODAL NEURAL NETWORK CONTROLLER
90a

| PROCESSOR 91 | MEMORY 92 | USER INTERFACE 93 |

SYSTEM BUS(ES)
96

STORAGE
95

CONTROL MODULES 97

DATA PREPROCESSOR
20
(*FIG. 3*)

ENCODED
NEURAL NETWORK
30
(*FIGS. 3, 4A & 4B*)

EMBEDDED
NEURAL NETWORK
40
(*FIGS. 3, 5A & 5B*)

SAMPLED
NEURAL NETWORK
50
(*FIGS. 3, 6A & 6B*)

CONVOLUTIONAL
NEURAL NETWORK
60
(*FIGS. 3 & 7*)

NETWORK
INTERFACE
94

# FIG. 9

FIG. 10

**Europäisches Patentamt**
**European Patent Office**
**Office européen des brevets**

# EUROPEAN SEARCH REPORT

Application Number

EP 18 16 4807

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEUNG BING LEUNG PATRICK ET AL: "Deep learning from electronic medical records using attention-based cross-modal convolutional neural networks", 2018 IEEE EMBS INTERNATIONAL CONFERENCE ON BIOMEDICAL & HEALTH INFORMATICS (BHI), IEEE, 4 March 2018 (2018-03-04), pages 222-225, XP033345164, DOI: 10.1109/BHI.2018.8333409 [retrieved on 2018-04-06] * abstract; figure 1 * * paragraph [000I] - paragraph [00II] * | 1-14 | INV. G06N3/04 |

TECHNICAL FIELDS SEARCHED (IPC)

G06N

The present search report has been drawn up for all claims

| Place of search | Date of completion of the search | Examiner |
|---|---|---|
| The Hague | 19 September 2018 | Cilia, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)